(19) Europäisches Patentamt
European Patent Office
Office européen des brevets

(11) Publication number: **0 031 388 B1**

(12) **EUROPEAN PATENT SPECIFICATION**

(45) Date of publication of patent specification: **07.03.84**

(51) Int. Cl.³: **C 07 D 233/64**

(21) Application number: **80901220.6**

(22) Date of filing: **04.07.80**

(86) International application number:
**PCT/JP80/00155**

(87) International publication number:
**WO 81/00109 22.01.81 Gazette 81/2**

(54) **PROCESS FOR PREPARING IMIDAZOLE DERIVATIVES.**

(30) Priority: **06.07.79 JP 86249/79**

(43) Date of publication of application:
**08.07.81 Bulletin 81/27**

(45) Publication of the grant of the patent:
**07.03.84 Bulletin 84/10**

(84) Designated Contracting States:
**DE FR GB**

(56) References cited:
**DE - A - 2 211 454**
**DE - A - 2 265 369**
**DE - A - 2 433 625**
**JP - A - 49 075 575**
**JP - A - 50 032 174**
**JP - A - 50 105 664**
**US - A - 4 128 658**

**March, Advances Organic Chemistry: Reactions, Mechanism, and structure, 1968, page 294**

(73) Proprietor: **SMITHKLINE BECKMAN CORPORATION**
**1 Franklin Plaza**
**Philadelphia Pennsylvania 19101 (US)**

(72) Inventor: **TERAJI, Tsutomu**
**20-6, Kofudai, 6-chome Toyono-cho**
**Toyono-gun, Osaka 563-01 (JP)**
Inventor: **NAKAI, Yoshiharu**
**7-18, Zeze 1-chome**
**Otsu-shi, Shiga 520 (JP)**
Inventor: **DURANT, Graham John**
**401 Knightsfield Welwyn Garden City**
**Hertfordshire (GB)**

(74) Representative: **Hargreaves, Gerald Henry, Dr. et al,**
**SMITH KLINE & FRENCH Laboratories Limited**
**Patent Department Mundells**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 031 388 B1

# Process for preparing imidazole derivatives

This invention relates to a process for preparing imidazole derivatives of the formula

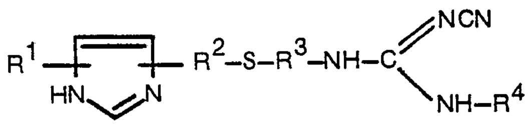 (I)

where $R^1$ and $R^4$ are each an alkyl group having 1 to 6 carbon atoms, and $R^2$ and $R^3$ are each an alkylene group having 1 to 6 carbon atoms, and their salts.

Imidazole derivatives of the formula (I) and their salts, which include cimetidine, are compounds having histamine $H_2$-antagonist activity and useful as anti-ulcer agents.

German Auslegeschriften 2,211,454 (pages 8—9) and 2,265,369 (Column 6, lines 39—58) describe the preparation of certain compounds of formula (I) in which a corresponding compound of the formula (II)

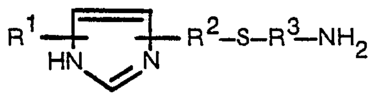 (II)

is reacted with a corresponding compound of the formula (III)

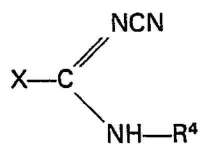 (III)

in which X is an alkoxy or a thioalkyl group, for instance a thiomethyl group, the leaving group X being displaced to form a guanidine. U.S. Patent 4,128,658 (column 5, lines 34—36) has a disclosure of the preparation of guanidine compounds by an analogous reaction between primary amines and certain compounds of formula (III) in which the leaving groups are halogen, thiomethyl, 3,5-dimethylpyrazolyl and alkoxy groups, but no method of preparation of such compounds in which the leaving groups are halogen and 3,5-dimethylpyrazolyl is disclosed, nor has any method for their preparation been described in any other publication prior to the present invention.

Methods for the preparation of compounds of formula (III) where X is halogen or an aromatic 5-membered N-containing heterocycle-N-yl group have now been found. It has been found that in these compounds and also in related compounds where X is a mercapto group or a group $—S(O)_nZ$ in which Z is hydroxy or alkyl, and n is 1 or 2, the group X also behaves as a leaving group in reactions with primary amines of formula (II), so that they provide new alternatives for the preparation of compounds of formula (I) by their reaction with amines of formula (II).

According to the invention, therefore, a process for preparing an imidazole derivative of formula (I) comprises reacting a corresponding compound of formula (II) with a corresponding compound of formula (III), characterised in that X is halogen, mercapto, an aromatic 5-membered N-containing heterocycle-N-yl group or a group $—S(O)_n—Z$ in which Z is hydroxy or alkyl, and n is 1 or 2.

In the starting materials each of $R^1$ and $R^4$ may be a straight or branched alkyl group having 1 to 6 carbon atoms such as methyl, ethyl, propyl, isopropyl, butyl, isobutyl, tert-butyl, pentyl or hexyl, and each of $R^2$ and $R^3$ may be a straight or branched alkylene group having 1 to 6 carbon atoms such as methylene, ethylene, trimethylene, tetramethylene, pentamethylene, hexamethylene, 2-methyltrimethylene or 2,2-dimethyltrimethylene. $R^2$ and $R^3$ may be the same or different from each other. When X is halogen, it may be chlorine, bromine or iodine. When X is an aromatic 5-membered N-containing heterocycle-N-yl group, it may be 1-pyrazolyl, 1-imidazolyl, 1-pyrrolyl, 1,2,3-triazol-1-yl, 3,5-dimethyl-1-pyrazolyl, or 1,2,4-triazol-4-yl. When X is a group $—S(O)_n—Z$, it may be sulfino, sulfo, alkylsulfinyl having 1 to 6 carbon atoms (e.g. methylsulfinyl, ethylsulfinyl, propylsulfinyl, isopropylsulfinyl, butylsulfinyl, isobutylsulfinyl, tert-butylsulfinyl, pentylsulfinyl and hexylsulfinyl), or alkylsulfonyl having 1 to 6 carbon atoms (e.g. methylsulfonyl, ethylsulfonyl, propylsulfonyl, isopropylsulfonyl, butylsulfonyl, isobutylsulfonyl, tert-butylsulfonyl, pentylsulfonyl and hexylsulfonyl).

The reaction can be carried out in a conventional solvent such as alcohol (e.g. methanol, ethanol, propanol, butanol and isopropyl alcohol), acetonitrile, methylene chloride, dimethylformamide, dimethylsulfoxide, dioxane and tetrahydrofuran. The reaction temperature is not critical and the reaction can be carried out at from room temperature to the boiling point of the solvent.

Where a compound (III) in which X is halogen or a sulfino or sulfo group is used as starting material, the reaction is preferably carried out in the presence of a base such as triethylamine, pyridine, N-methylaniline and 1,5-diazabicyclo[5.4.0]undec-5-ene.

Where a compound (III) in which X is a mercapto group is used as starting material, the reaction is preferably carried out in the presence of a compound of a heavy metal (e.g. mercury, silver and lead).

Where a compound (III) in which X is a sulfino or sulfo group is used, it can be in the form of a salt such as a salt of an alkali metal (e.g. sodium and potassium) or a 1,5-diazabicyclo[5.4.0]undec-5-ene (DBU) salt.

The imidazole derivative (I) produced by a process of this invention may be isolated and purified, and if desired, converted into its salt such as a hydrochloride in a conventional manner.

The starting materials used in the process of this invention can be prepared by methods described in the following Examples or by similar methods.

The following Examples illustrate the preparation of cimetidine by the process of the invention.

Example 1

(i) To a stirred suspension of N-cyano-N′,S-dimethylisothiourea (60 g) in chloroform (600 ml) was added a solution of chlorine (82.6 g) in chloroform (500 ml) over 30 min at 0—5°C. After stirring for 1.5 hr at the same temperature, the solvents were evaporated under reduced pressure and the residue was washed with dry benzene and dried in vacuum. The resulting white powder was dissolved in hot dry acetone (250 ml). After filtration of the insoluble impurities, the filtrate was passed through a short column of silica (60 g) and eluted with methylene dichloride. The eluate was dried up under reduced pressure and the residue was recrystallised from acetone-light petroleum to give N-cyano-N′-methylchloroformamidine (36.8 g), yield 67.4%.

The product was recrystallised twice from benzeneacetone to obtain white crystals, m.p. 130.5—131.5°C.

(Calc. for $C_3H_4ClN_3$: C, 30.60; H, 3.43; N, 35.75; Cl, 30.16. Found: C, 30.69; H, 3.25; N, 35.63; Cl, 29.82).

IR (nujol) $\nu$ max: 3240, 3020, 2220, 1625, 1540, 1403, 1318, 1272, 1257, 1172, 1095, 1048, 1020, 733, 722 $cm^{-1}$.

H—NMR ($CD_3OD$) $\delta$ 2.92 (s, 3H).

(ii) To a stirred solution of 2-(5-methylimidazol-4-ylmethylthio)ethylamine (1.0 g) and triethylamine (0.71 g) in methanol (20 ml) was added N-cyano-N′-methylchloroformamidine (0.82 g) and the mixture was stirred for 7.5 hr at 50—55°C. After evaporation, the residue was subjected to chromatography on silica [eluant: $CHCl_3$—MeOH (10:1)]. The resulting crude product was added to water (3 ml) and extracted repeatedly with ethyl acetate. The extracts were dried over anhydrous magnesium sulfate and concentrated under reduced pressure to small volume. The resulting crystals were collected by filtration and washed with ethyl acetate to give N-cyano-N′-methyl-N″-[2-(5-methylimidazol-4-ylmethylthio)ethyl]guanidine (0.33 g), yield 22.4%, m.p. 134—137°C.

Example 2

A suspension of N-cyano-N′-methylthiourea (DBU salt) (0.534 g), 2-(5-methylimidazol-4-ylmethylthio)ethylamine (1.03 g), mercuric oxide (0.868 g) and anhydrous sodium sulfate (2 g) in acetonitrile (20 ml) was heated under reflux for 20 hr with vigorous stirring. The hot reaction mixture was filtered and the filtrate was dried up and subjected to chromatography on silica [eluant: $CH_3CN$—$CH_3OH$ (4:1)]. The resulting crude product was purified by preparative thin layer chromatography [$SiO_2$, AcOEt—MeOH—28% aq. $NH_3$ (10:1:1)] and recrystallised from acetonitrile to give N-cyano-N′-methyl-N″-[2-(5-methylimidazol-4-ylmethylthio)ethyl]guanidine (38.4 mg), yield 7.6%, m.p. 136—138°C.

Example 3

(i) To a stirred suspension of N-cyano-N′,S-dimethyliso-thiourea (19.4 g) in acetone (700 ml) was added dropwise a solution of *m*-chloroperbenzoic acid (80% purity, 38.8 g) in acetone (120 ml) at 20—25°C. After stirring for 1 hr at the same temperature, the reaction mixture was cooled in an ice bath for 1 hr and the precipitate was filtered and washed with acetone to give N-cyano-N′-methyl-methyl-sulfinylformamidine (13.65 g), yield 62.8%, m.p. 115.5—116°C.

(Calc. for $C_4H_7N_3OS$: C, 33.09; H, 4.86; N, 28.94; S, 22.07. Found: C, 33.08; H, 4.78; N, 28.79; S, 22.14).

IR (nujol) $\nu$ max: 3240, 2200, 1621, 1512, 1418, 1393, 1282, 1173, 1073, 1033, 883, 743 $cm^{-1}$.

NMR (DMSO-$d_6$): $\delta$ 2.82 (s), 2.84 (s).

A small crop of the same product (2.60 g, m.p. 112—112.5°C was obtained from the filtrate.

(ii) A solution of N-cyano-N′-methyl-methylsulfinylformamidine (493 mg) and 2-(5-methylimidazol-4-ylmethylthio)ethylamine (684 mg) in acetonitrile (20 ml) was stirred for 4 hr at room temperature. After concentration under reduced pressure, the residue was subjected to chromatography on silica [eluant: $CH_3CN$—$CH_3OH$ (4:1)]. The product obtained was recrystallised from acetonitrile to give N-cyano-N′-methyl-N″-[2-(5-methylimidazol-4-yl-methylthio)-ethyl]guanidine (684 mg), yield 79.7%, m.p. 136—138°C.

Example 4

(i) To a stirred suspension of sodium hydride (50% oil suspension, 3.58 g) in tetrahydrofuran (160 ml) was added pyrazole (5.84 g) at a temperature below room temperature and stirred for 1 hr. To the mixture was added N-cyano-N′-methyl-methylsulfinylformamidine (8.32 g, obtained as in Example 3(i)) and stirred for 3 hr at room temperature. After evaporation under reduced pressure, the residue was dissolved in water and washed with ether. The aqueous layer was acidified (pH 4) with 10% hydrochloric acid under ice-cooling. The precipitate was filtered, washed with water and dried to give 1-(N-cyano-N′-methylamidino)pyrazole (7.52 g). An additional amount (0.2 g) of the same product was obtained from the filtrate. The crude products were combined and recrystallised from ethyl acetate to give the purified product (6.12 g), yield 71.7%, m.p. 166.5—168°C.
(Calc. for $C_6H_7N_5$: C, 48.31; H, 4.73; N, 46.96. Found: C, 48.40; H, 4.69; N, 46.81).
IR (nujol) $\nu$ max: 3280, 2200, 1660, 1645, 1540, 1421, 1387, 1348, 1268, 1207, 1173, 1084, 1040, 985, 922, 908, 838, 765, 702 $cm^{-1}$.
NMR (DMSO-$d_6$): $\delta$ 3.13 (s, 3H), 6.65 (dd, J=2Hz, J=3Hz, 1H), 7.95 (d, J=2Hz, 1H), 8.53 (d, J=3Hz, 1H).

(ii) A solution of 1-(N-cyano-N′-methylamidino)pyrazole obtained as above (373 mg) and 2-(5-methylimidazol-4-ylmethylthio)ethylamine (503 mg) in acetonitrile (15 ml) was heated under reflux for 24 hr. The reaction mixture was concentrated under reduced pressure and the residue was extracted to chromatography on silica [eluant: $CH_3CN$—$CH_3OH$ (4:1)]. The crude product obtained was recrystallised from acetonitrile to give N-cyano-N′-methyl-N″-[2-(5-methylimidazol-4-ylmethylthio)-ethyl]guanidine (359 mg), yield 56.9%, m.p. 137—138.5°C.

Example 5

(i) N-cyano-N′-methylthiourea (DBU salt) (10 g) in water (20 ml) was added to a solution of 35% hydrogen peroxide (21.8 g) and sodium tungstate dihydrate (0.1 g) in water (40 ml) under ice-cooling. After stirring at room temperature for 1.5 hr, the precipitate was filtered off. The filtrate was concentrated under reduced pressure and purified by column chromatography [HP—20 resin (Mitsubishi Chem. Ind. Ltd.)] to obtain the DBU salt of cyanoimino-methylaminomethanesulfonic acid (1.5 g, 12.7%). The product was further recrystallised from isopropyl alcohol to give the purified DBU salt of cyanoimino-methylaminomethanesulfonic acid, m.p. 135—136°C.
(Calc. for $C_{12}H_{21}N_5O_3S$: C, 45.70; H, 6.71; N, 22.21; S, 10.16. Found: C, 45.82; H, 6.71; N, 22.20; S, 10.12).
IR (nujol) $\nu$ max: 3290, 3160, 2190, 1650, 1608, 1523, 1460, 1415, 1380, 1325, 1263, 1232, 1215, 1203, 1105, 1060, 1028, 1003, 727 $cm^{-1}$.

(ii) A solution of cyanoimino-methylaminomethanesulfonic acid (DBU salt) (0.70 g) and 2-(5-methylimidazol-4-ylmethylthio)ethylamine (0.57 g) in ethanol (14 ml) was heated under reflux for 7 hr. After removal of the solvent under reduced pressure, the residue was purified by column chromatography on $SiO_2$ [eluant: $CH_3CN$—$CH_3OH$ (4:1)]. After concentration of the eluate under reduced pressure, the residue was subjected to preparative thin layer chromatography [$SiO_2$, $CH_3COOCH_3$—$CH_3OH$—28% aq. $NH_3$ (10:1:1)] and then recrystallised from $CH_3OH$—$CH_3CN$ to give N-cyano-N′-methyl-N″-[2-(5-methylimidazol-4-ylmethylthio)ethyl]-guanidine (0.18 g), yield 32.4%, m.p. 140—141°C.

## Claims

1. A process for preparing an imidazole derivative of the formula

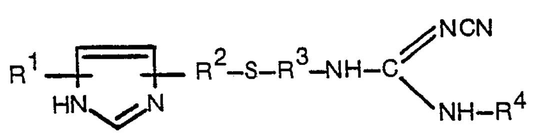

(I)

where $R^1$ and $R^4$ are each an alkyl group having 1 to 6 carbon atoms, and $R^2$ and $R^3$ are each an alkylene group having 1 to 6 carbon atoms, which comprises reacting a corresponding compound of the formula

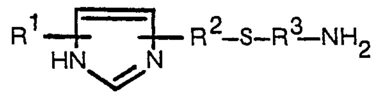

(II)

with a compound of the formula

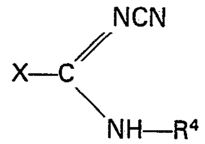

characterised in that X is halogen, mercapto, an aromatic 5-membered N-containing heterocycle-N-yl group or a group —$S(O)_n$—Z in which Z is hydroxy or alkyl, and n is 1 or 2.

2. A process according to Claim 1, in which the imidazole derivative is cimetidine.

**Revendications**

1. Procédé de préparation d'un dérivé d'imidazole de formule:

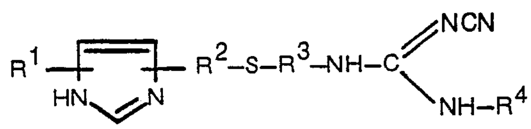

dans laquelle $R^1$ et $R^4$ représentent chacun un groupe alcoyle ayant de 1 à 6 atomes de carbone et $R^2$ et $R^3$ représentent chacun un groupe alcoylène ayant de 1 à 6 atomes de carbone, qui comprend la mise en réaction d'un composé correspondant de formule:

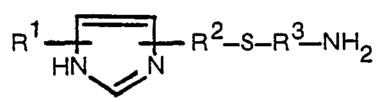

avec un composé de formule:

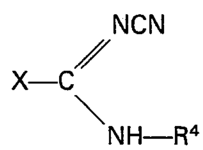

caractérisée en ce que X représente un halogène, un groupe mercapto, un groupe hétérocycle-N-yle aromatique pentagonal contenant de l'azote ou un groupe —$S(O)_n$—Z dans lequel Z — représente un groupe hydroxy ou alcoyle et n est égal à 1 ou 2.

2. Procédé suivant la revendication 1, dans lequel le dérivé d'imidazole est la cimétidine.

**Patentansprüche**

1. Ein Verfahren zur Herstellung eines Imidazolderivats der Formel

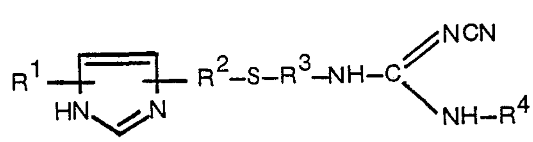

in der $R^1$ und $R^4$ je eine Alkylgruppe mit 1 bis 6 Kohlenstoffatomen und $R^2$ und $R^3$ je eine Alkylengruppe mit 1 bis 6 Kohlenstoffatomen bedeuten, dadurch gekennzeichnet, daß man eine entsprechende Verbindung der Formel

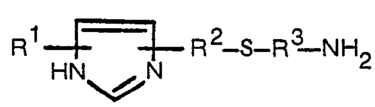

mit einer Verbindung der Formel

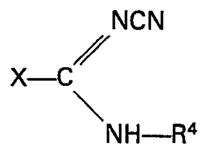

umsetzt, wobei X Halogen, Mercapto, eine aromatische, 5-gliedrige, N-enthaltende Heterocyclus-N-yl-Gruppe oder den Rest —$S(O)_n$—Z bedeutet, in dem Z Hydroxy oder Alkyl ist und n 1 oder 2 bedeutet.

2. Ein Verfahren nach Anspruch 1, wobei das Imidazolderivat Cimetidin ist.